# EUROPEAN PATENT APPLICATION

(11) **EP 4 599 793 A1**
(43) Date of publication of application: **13.08.2025**
(21) Application number: 23875045.9
(22) Date of filing: 22.08.2023
(51) Int. Cl.: A61C 7/00, A61C 5/70, A61C 9/00, G16H 50/50, G16H 30/00, A61C 7/08

(54) **METHOD, DEVICE, AND RECORDING MEDIUM FOR PROVIDING INFORMATION FOR ORTHODONTIC ATTACHMENT**

(30) Priority: 07.10.2022 KR 20220128418
(71) Applicant: Osstem Implant Co., Ltd., Gangseo-gu Seoul 07789 (KR)
(72) Inventor: CHOI, Kyoo Ok, Seoul 07789 (KR); KIM, Hwa Sam, Seoul 07789 (KR)
(74) Representative: Klunker IP Patentanwälte PartG mbB
(86) International application number: PCT/KR2023/012438
(87) International publication number: WO 2024/075975

(57) **Abstract**

Disclosed are a method, a device, and a recording medium according to an embodiment, the method including the steps in which: a reception part acquires a pre-orthodontic tooth model indicating a pre-orthodontic state of a tooth model to which a virtual checker is attached; a processor acquires a post-orthodontic tooth model indicating a post-orthodontic state of the tooth model; the processor acquires orthodontic force data on the basis of a comparison result of the pre-orthodontic tooth model and the post-orthodontic tooth model; the processor acquires attachment-related information including information on whether an attachment is required for orthodontic treatment, on the basis of checker orthodontic force data indicating orthodontic force corresponding to the checker, which is acquired from the orthodontic force data; and an output part displays the attachment-related information.

## Description

### [Technical Field]

The present disclosure relates to a technology for providing information for an orthodontic attachment, and more specifically, to a technology field for providing attachment-related information on the basis of orthodontic force required for tooth movement using a clear aligner.

### [Background Art]

Various methods have traditionally been developed and used to move teeth to desired positions for orthodontic purposes.

One of these methods is a fixed orthodontic treatment method that is based on the principle of attaching a device (e.g., bracket) to a tooth and applying force (e.g., the elasticity of wires) to gradually move the tooth. However, this method has a drawback of causing discomfort due to the prolonged fixation of multiple devices in the patient's oral cavity.

To address the issues of fixed orthodontic treatment methods, a removable orthodontic treatment approach has been introduced, which moves teeth by wearing a transparent device, such as a mouthpiece, on the teeth instead of using the elasticity of wires attached to brackets fixed to the teeth. This removable treatment method has dramatically reduced the discomfort associated with traditional fixed treatment by making it easy for patients to attach and remove the device.

According to the removable treatment method, the teeth is covered with a transparent device that directly applies force to push the teeth toward their desired positions. If the force applied by the device to directly push the teeth is insufficient, it is difficult to move the teeth to the desired positions, which is a limitation of this approach.

To overcome this limitation, the removable treatment method addresses the shortcomings of traditional approaches by applying attachments with specific shapes to the tooth surface. However, the method of using attachments based on user experience presents challenges, including difficulties in uniformly producing attachments in sets or in a lump, misuse of attachments impeding expected tooth movement, and excessive use of attachments reducing the convenience of removable orthodontic treatment.

### [Disclosure]

### [Technical problem]

An embodiment of the present disclosure, which aims to address the aforementioned issues of the related art, may provide a method, a device, and a computer program stored in a recording medium for providing attachment-related information for orthodontic treatment by using orthodontic force data obtained based on pre- and post-orthodontic models of a tooth model.

According to an embodiment of the present disclosure, the technical objective includes enhancing the orthodontic effect achieved with clear aligners by obtaining and analyzing orthodontic force data, which varies for each tooth, to provide attachment-related information.

The technical objectives to be addressed are not limited to those described above, and may further include various technical objectives that would be apparent to those of ordinary skill in the art.

### [Technical Solution]

A method performed by a device for providing information for an orthodontic attachment according to a first aspect of the present disclosure includes the steps of: acquiring, at a reception part, a pre-orthodontic tooth model indicating a pre-orthodontic state of a tooth model to which a virtual checker is attached; acquiring, at a processor, a post-orthodontic tooth model indicating a post-orthodontic state of the tooth model; acquiring, at the processor, orthodontic force data based on a comparison result of the pre-orthodontic tooth model to the post-orthodontic tooth model; acquiring, at the processor, attachment-related information including information on whether an attachment is required for orthodontic treatment, based on checker orthodontic force data, which represents orthodontic force corresponding to the checker and is acquired from the orthodontic force data; and displaying, at an output part, the attachment-related information.

In one embodiment, the step of acquiring the attachment-related information may include determining whether the attachment is necessary based on the presence or absence of the orthodontic force corresponding to the checker.

In one embodiment, the step of acquiring the attachment-related information may include the steps of: acquiring crown orthodontic force data from the orthodontic force data, wherein the crown orthodontic force data represents orthodontic force corresponding to a crown of the tooth model that includes the crown and the checker,; and acquiring the attachment-related information using both the checker orthodontic force data and the crown orthodontic force data.

In one embodiment, the step of acquiring the attachment-related information using both the checker orthodontic force data and the crown orthodontic force data may include the steps of: determining checker orthodontic force, which is orthodontic force corresponding to the checker, based on the checker orthodontic force data; determining crown orthodontic force, which is orthodontic force corresponding to the crown, based on the crown orthodontic force data; and determining whether the attachment is necessary based on whether the checker orthodontic force corresponds to the crown orthodontic force.

In one embodiment, the step of determining whether the attachment is necessary based on whether the orthodontic force corresponds to the crown orthodontic force may include determining that the attachment is unnecessary when the checker orthodontic force is substitutable with the crown orthodontic force, and determining that the attachment is necessary when the checker orthodontic force is not substitutable with the crown orthodontic force.

In one embodiment, the step of acquiring the attachment-related information may include the steps of: determining a direction of the orthodontic force based on the magnitude and distribution of the force corresponding to the checker; and acquiring the attachment-related information based on the direction of the orthodontic force.

In one embodiment, the step of acquiring the attachment-related information may include determining whether the attachment is necessary based on whether there is orthodontic force corresponding to an edge-adjacent region of the checker having an angular shape.

In one embodiment, the checker may be attached to the buccal or labial side of a crown and have a rectangular shape including a first edge-adjacent region to a fourth edge-adjacent region, and the step of acquiring the attachment-related information may include the steps of: determining a direction of the orthodontic force to be at least one of a distal direction, a mesial direction, a cervical direction, or an occlusal direction based on a region to which the orthodontic force corresponds, among the first edge-adjacent region to the fourth edge-adjacent region; and acquiring the attachment-related information based on the direction of the orthodontic force.

In one embodiment, the step of acquiring the orthodontic force data may include determining a region to which the orthodontic force corresponds and the magnitude of the orthodontic force based on the degree of separation between the pre-orthodontic tooth model and the post-orthodontic tooth model when they are matched.

In one embodiment, the orthodontic force data may include data on the magnitude of the orthodontic force and data on the distribution of the orthodontic force.

A device for providing information for an orthodontic attachment according to a second aspect of the present disclosure includes: a reception part configured to acquire a pre-orthodontic tooth model indicating a pre-orthodontic state of a tooth model to which a virtual checker is attached and a post-orthodontic tooth model indicating a post-orthodontic state of the tooth model; a processor configured to acquire orthodontic force data based on a comparison result of the pre-orthodontic tooth model to the post-orthodontic tooth model and acquire attachment-related information including information on whether an attachment is required for orthodontic treatment, based on checker orthodontic force data, which represents orthodontic force corresponding to the checker and is acquired from the orthodontic force data; and an output part configured to display the attachment-related information.

In one embodiment, the processor may acquire crown orthodontic force data from the orthodontic force data, wherein the crown orthodontic force data represents orthodontic force corresponding to a crown of the tooth model that includes the crown and the checker, and acquire the attachment-related information using both the checker orthodontic force data and the crown orthodontic force data.

In one embodiment, the processor may determine checker orthodontic force, which is orthodontic force corresponding to the checker, based on the checker orthodontic force data, determine crown orthodontic force, which is orthodontic force corresponding to the crown, based on the crown orthodontic force data, and determine whether the attachment is necessary based on whether the checker orthodontic force corresponds to the crown orthodontic force.

In one embodiment, the processor may determine that the attachment is unnecessary when the checker orthodontic force is substitutable with the crown orthodontic force, and determine that the attachment is necessary when the checker orthodontic force is not substitutable with the checker orthodontic force.

In one embodiment, the processor may determine whether the attachment is necessary based on whether there is orthodontic force corresponding to an edge-adjacent region of the checker having an angular shape.

According to a third aspect of the present disclosure, there is provided a computer-readable recording medium having recorded thereon a program which, when executed by a computer, causes the computer to perform the method of the first aspect.

Other specific details are included in the detailed description and drawings.

### [Effects of the Invention]

According to one embodiment, information related to an attachment required for orthodontic treatment using clear aligners can be automatically provided by utilizing orthodontic force acquired through a comparison of pre- and post-orthodontic tooth models.

Additionally, since the determination of whether to use an attachment during clear aligner treatment is provided through system calculations based on orthodontic force, the accuracy of the treatment plan can be improved.

Moreover, by providing the basis for whether an attachment is necessary, along with the orthodontic results achieved using the attachment, and allowing the user to review this information, the user can more easily make decisions regarding the necessity of the attachment.

In addition, the indications for orthodontic treatment using clear aligners can be improved.

Additionally, minimizing user dependence on determining whether to use an attachment during clear aligner treatment can reduce the time required for treatment planning.

It should be understood that the effects of the present disclosure are not limited to the aforementioned effects and include all effects that can be inferred from the configurations of the invention described in the detailed description or the claims.

### [Description of Drawings]

FIG. 1 is a schematic diagram illustrating an example configuration of a device according to one embodiment.
FIG. 2 is a flowchart illustrating each step performed by a device to display attachment-related information according to one embodiment.
FIG. 3 schematically illustrates an example in which an attachment example model is displayed according to one embodiment.
FIG. 4 schematically illustrates an example in which whether an attachment is required for orthodontic treatment is determined based on the orthodontic force applied in each direction to a checker and crown according to one embodiment.
FIGS. 5 to 12 schematically illustrate examples in which whether an attachment is necessary is determined based on the orthodontic force applied in each direction to a checker and a crown according to one embodiment.

### [Mode of the Invention]

The terms used in the embodiments are those general terms currently widely used in the art in consideration of functions in regard to the inventive concept, but the terms may vary according to the intention of those of ordinary skill in the art, precedents, or new technology in the art. Also, specified terms may be selected by the applicant, and in this case, the detailed meaning thereof will be described in the detailed description of the inventive concept. Thus, the terms used in the specification should be understood not as simple names but based on the meaning of the terms and the overall description of the inventive concept.

Throughout the specification, it will also be understood that when a component "comprises," or "includes" an element, unless there is another opposite description thereto, it should be understood that the component does not exclude another element but may further include another element. Furthermore, the term "unit (or part)" used in the specification refers to a unit for processing at least one function or operation, and this may be realized in the form of hardware, software, or in a combination of both hardware and software.

Embodiments of the present disclosure will now be described in detail with reference to the accompanying drawings so that those skilled in the art can easily practice the embodiments of the present disclosure. However, the present disclosure may be implemented in many different forms and is not limited to the embodiments described herein.

The present disclosure provides various embodiments for deriving pre- and post-treatment orthodontic forces in orthodontic treatment using a clear aligner and for providing attachment-related information, including the necessity of an attachment during orthodontic treatment using a clear aligner, based on the derived orthodontic forces.

Hereinafter, a plurality of embodiments of the present disclosure will be described in detail with reference to the drawings.

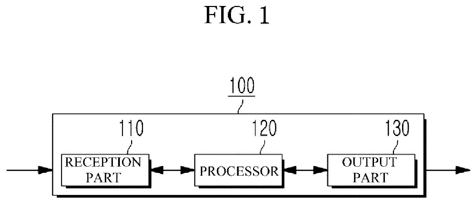

FIG. 1 is a schematic diagram illustrating an example configuration of a device 100 according to one embodiment.

Referring to FIG. 1, the device 100 may include a reception part 110, a processor 120, and an output part 130.

The reception part 110 according to one embodiment may acquire a pre-orthodontic tooth model representing a pre-orthodontic state of a tooth model to which a virtual checker, which is an attachment-type device, is attached, and a post-orthodontic tooth model representing a post-orthodontic state of the tooth model in order to evaluate orthodontic force. The checker is an object attached to the tooth model to determine the magnitude and direction of the orthodontic force applied to a crown of the tooth model during orthodontic treatment using a clear aligner.

In one embodiment, the reception part 110 may acquire a pre-orthodontic tooth model without the virtual checker attached, as well as a tooth model representing a post-orthodontic state. Based on the acquired pre-orthodontic and post-orthodontic tooth models, the reception part 110 may further determine whether the tooth have moved, along with the distance and direction of movement. Alternatively, the processor 120 may compare the pre-orthodontic and post-orthodontic tooth models to quantify the difference and classify the magnitude of the quantified difference information. Here, the quantified difference information may be converted into a designated color and displayed on the pre-orthodontic tooth model.

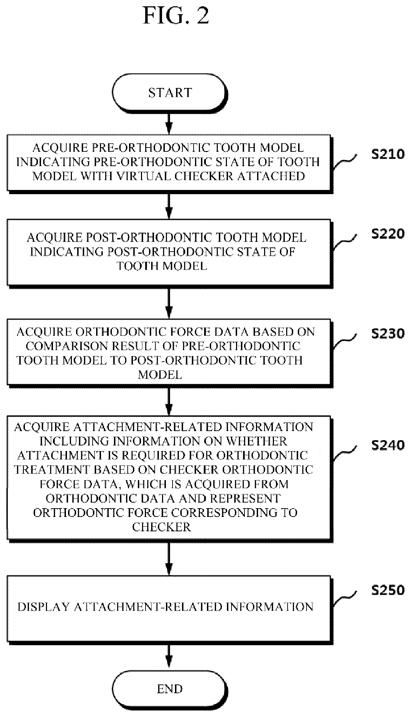

Then, the processor 120 may obtain a tooth model in which a virtual checker is selectively attached to the tooth from which the quantified difference information is acquired, and may apply the orthodontic force obtained according to the embodiments described below to the virtual checker. The above-described embodiment will be described below with reference to FIGS. 2 to 12.

The reception part 110 may receive information provided by an external device 100. For example, the reception part 110 may include a wired or wireless communication device capable of receiving various types of information by connecting to the external device 100 or external components (not shown) through a network or signal processing module.

In one embodiment, the checker may have an angular shape. For example, the checker may be of a rectangular shape including a first edge-adjacent region to a fourth edge-adjacent region. Additionally, the checker may be displayed on the tooth model in a form attached to the labial or buccal surface of the crown, aligned with the tooth axis of the crown.

In one embodiment, the tooth model may include modeling data for the teeth of a patient undergoing orthodontic treatment using a clear aligner. Additionally, the pre-orthodontic tooth model may represent the state of the teeth before the start of orthodontic treatment with the clear aligner, while the post-orthodontic tooth model may represent the expected state of the teeth once the desired tooth movement has been completed through the orthodontic treatment using the clear aligner. Furthermore, the tooth model may include not only the pre-orthodontic and post-orthodontic tooth models for the entire orthodontic process using the clear aligner but also the pre-orthodontic and post-orthodontic tooth models for any specific stage of the orthodontic process, and may be displayed by the device 100.

Additionally, the pre-orthodontic and post-orthodontic tooth models may show differing crown positions due to the tooth movement caused by the orthodontic treatment.

The processor 120 according to one embodiment may acquire orthodontic force data representing the orthodontic force required for the orthodontic treatment, based on the comparison result of the pre-orthodontic tooth model to the post-orthodontic tooth model.

For example, the orthodontic force data may be acquired based on the comparison of the extent to which the crown has moved between the pre-orthodontic and post-orthodontic tooth models. Moreover, the orthodontic force data may include crown orthodontic force data that represents the orthodontic force acting on the crown included in the tooth model along with the checker, and checker orthodontic force data that represents the orthodontic force acting on the checker.

In one embodiment, the processor 120 may acquire attachment-related information, including information on whether an attachment is required for orthodontic treatment, using both the checker orthodontic force data, which is obtained from the orthodontic force data and represents the orthodontic force corresponding to the checker, and the crown orthodontic force data.

For example, the processor 120 may determine whether the attachment is necessary based on whether the checker orthodontic force derived from the checker orthodontic force data corresponds to the crown orthodontic force data included in the orthodontic force data.

Specifically, the processor 120 may determine that the attachment is unnecessary if the checker orthodontic force can be substituted with the crown orthodontic force and may determine that the attachment is necessary if the checker orthodontic force cannot be substituted with the crown orthodontic force.

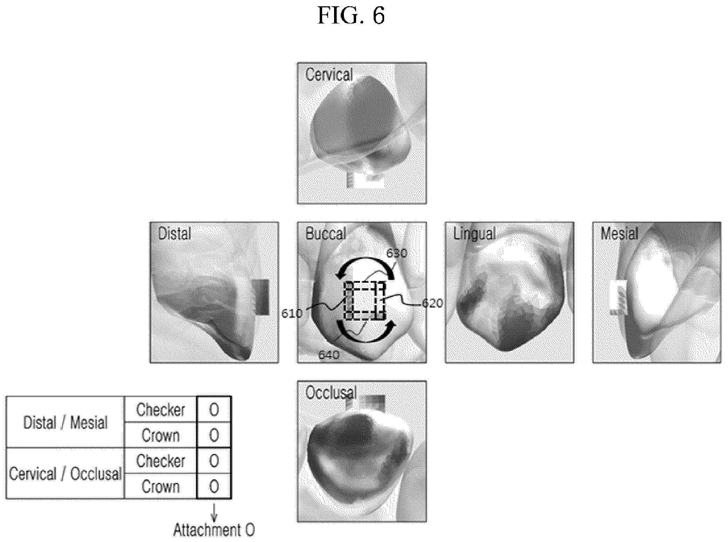

In one embodiment, the processor 120 may determine whether the attachment is necessary based on whether there is an orthodontic force corresponding to the edge-adjacent region of the checker. Referring to FIG. 6, for example, the edge-adjacent regions of the checker, viewed from the buccal perspective, may include a distal region 610, a mesial region 620, a cervical region 630, and an occlusal region 640.

In one embodiment, the output part 130 may display the attachment-related information.

The embodiments described above will be further explained in more detail with reference to FIGS. 2 to 12.

Additionally, the processor 120 may perform a series of operations to acquire the attachment-related information and may be electrically connected to other components, not shown, in addition to the reception part 110 and the output part 130, to control data flow among them. For this purpose, the processor 120 may be implemented as a central processing unit (CPU) that controls the overall operation of the device 100.

In addition, it should be understood by those skilled in the art that, in addition to the components shown in FIG. 1, other general components may be further included in the device 100. According to one embodiment, the device 100 may further include a user interface part for receiving user input and a storage part configured to store data described throughout the specification.

FIG. 2 is a flowchart illustrating each step performed by the device 100 to display the attachment-related information according to one embodiment.

According to one embodiment, the attachment may be used during the orthodontic process to increase the orthodontic force applied to the tooth. Additionally, the attachment-related information may broadly refer to information associated with the attachment. For example, the attachment-related information may indicate whether an attachment is required for orthodontic treatment. In one example, in case that the orthodontic force required for orthodontic treatment is smaller than a preset value, an attachment may not be necessary; however, in case that the orthodontic force exceeds the preset value, an attachment may be required.

Referring to FIG. 2, in steps S210 and S220, the device 100 may acquire a pre-orthodontic tooth model representing the pre-orthodontic state of a tooth model with a virtual checker attached, and a post-orthodontic tooth model representing the post-orthodontic state of the tooth model with the virtual checker attached.

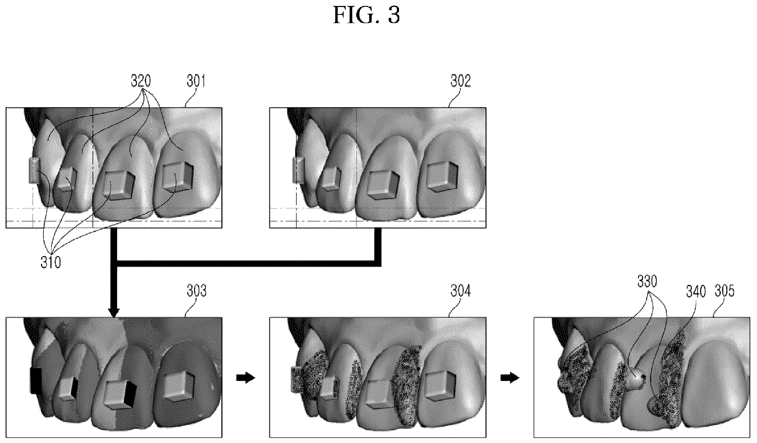

Referring to FIG. 3, the pre-orthodontic tooth model 301 and the post-orthodontic tooth model 302 may be displayed in the form with a virtual checker 310 attached, as shown in the drawing. The post-orthodontic tooth model 302 may also be displayed, as shown in the drawing, indicating the movement of crown 320 and the checker 310 from their pre-orthodontic positions as a result of orthodontic treatment.

Specifically, the crown 320 and the checker 310 displayed on the post-orthodontic tooth model 302 may be displayed as having moved from their positions on the pre-orthodontic tooth model 301 to correspond to the movement distance of the crown 320 and the checker 310 during orthodontic treatment. In another embodiment, the checker 310 displayed on the post-orthodontic tooth model 302 may be displayed as having moved to correspond to the movement distance of the crown 320 during orthodontic treatment.

In yet another embodiment, the pre-orthodontic tooth model 301 and the post-orthodontic tooth model 302 may represent the state of the tooth models before and after orthodontic treatment without the checker 310 being attached. In this case, the checker 310 displayed on the pre-orthodontic tooth model 301 may be displayed on the crown 320 in a manner that corresponds to the position and tooth axis of the crown 320. The post-orthodontic tooth model 302 may be displayed with the checker 310 moved to a position corresponding to the movement distance of the crown 320 during orthodontic treatment.

In another embodiment, the device 100 may generate and/or place the checker 310 on each of the pre-orthodontic tooth model 301 and the post-orthodontic tooth model 302. The checker 310 may be positioned on the shape of the crown parallel to the tooth axis of each crown and may specifically be placed so that the direction of the checker aligns with the direction of the crown. Here, the direction of the crown may be distinguished or determined as buccal, distal, mesial, cervical, or occlusal direction, and the direction of the checker 310 may be determined from the stage at which the checker 310 is generated or placed. Additionally, the position of the checker 310 may be placed in the same location on both the pre-orthodontic tooth model 301 and the post-orthodontic tooth model 302.

The pre-orthodontic tooth model 301 and post-orthodontic tooth model 302 illustrated in FIG. 3 only show some crowns 320 and checkers 310. However, this is for the purpose of providing a more detailed explanation of the embodiments and is not necessarily limited to the drawings. The embodiments described with reference to FIG. 3 can equally apply in cases where the entire crowns 320 and checkers 310 are shown.

In step S230, the device 100 may acquire orthodontic force data 304, representing the orthodontic force required for orthodontic treatment, based on the comparison between the pre-orthodontic tooth model 301 and the post-orthodontic tooth model 302.

In one embodiment, the orthodontic force may refer to the pushing force required to move the crown 320 to a desired position.

In one embodiment, as shown in FIG. 3, the comparison result of the pre-orthodontic tooth model 301 to the post-orthodontic tooth model 302 may be obtained based on the degree of separation of each crown 320 and checker 310 appearing on a matched model 303, which is generated by matching the pre-orthodontic tooth model 301 and the post-orthodontic tooth model 302 based on a preset reference point. The device 100 may determine the region to which the orthodontic force corresponds and the magnitude of the orthodontic force based on the comparison result. According to one embodiment, the region to which the orthodontic force corresponds may represent the area where the orthodontic force is applied. For example, a region where the orthodontic force is applied at or above a preset proportion may correspond to the region for that orthodontic force.

For instance, the device 100 may display the region to which the orthodontic force corresponds and the movement distance between the pre-orthodontic tooth model 301 and the post-orthodontic tooth model 302 with a color map, based on the degree of separation of each crown 320 and checker 310 on the post-orthodontic tooth model 302 that appears on the matched model 303. The device 100 may acquire the orthodontic force data 304, including data on movement distance and the distribution of orthodontic force, based on the region, distribution, and color of the color map displayed on the crown 320 and the checker 310. The region, distribution, and colors of the color map may be determined based on preset units (e.g., pixels).

In one embodiment, when the color map represents information on movement distance, different colors may be displayed for different movement distances. For example, if the movement distance falls within a preset first range (e.g., 0 mm to less than 0.1 mm), the color of the region corresponding to the movement distance may be displayed as a first color (e.g., yellow). If the movement distance falls within a preset second range (e.g., greater than 0.1 mm to less than 0.2 mm), the color of the region corresponding to the movement distance may be displayed as a second color (e.g., orange). If the movement distance falls within a preset third range (e.g., greater than 0.2 mm to less than N mm), the color of the region corresponding to the movement distance may be displayed as a third color (e.g., red).

In another embodiment, the colors on the color map may be determined based on the magnitude of the orthodontic force.

Specifically, the region to which the orthodontic force corresponds may be determined to correspond to the region and distribution of the color map, and the intensity or lightness of the color on the color map may represent the magnitude of the orthodontic force. For example if the color map is distributed over a first region of a first crown 340 and the intensity of the color in the first region indicates a first level, the device 100 may determine the region corresponding to the orthodontic force based on the first region, and the magnitude of the orthodontic force based on the first level.

In one embodiment, the device 100 may acquire data on the magnitude of the orthodontic force and data on the distribution of the orthodontic force based on the region corresponding to the orthodontic force and the magnitude of the orthodontic force. Additionally, data on the magnitude of the orthodontic force and data on the distribution of the orthodontic force may be included in the orthodontic force data 304.

In step S240, the device 100 may acquire attachment-related information, including information on whether an attachment is required for orthodontic treatment, based on the checker orthodontic force data. The checker orthodontic force data may be obtained from the orthodontic force data 304 and may represent the orthodontic force corresponding to the checker 310.

Specifically, the device 100 may acquire the attachment-related information based on crown orthodontic force data representing the orthodontic force. The orthodontic force may be obtained from the checker orthodontic force data and the orthodontic force data 304. Additionally, the orthodontic force may correspond to the crown 320.

For example, referring to FIG. 3, the checker orthodontic force data may be obtained based on the data on the magnitude of the orthodontic force and the data on the distribution of the orthodontic force which are acquired from the checker 310 and included in the orthodontic force data 304. The crown orthodontic force data may be obtained based on the data on the magnitude of the orthodontic force and the data on the distribution of the orthodontic force which are included in the orthodontic force data 304.

Additionally, the device 100 may determine checker orthodontic force, which is the orthodontic force corresponding to the checker 310, based on the checker orthodontic force data, and determine crown orthodontic force, which is the orthodontic force corresponding to the crown 320, based on the crown orthodontic force data. The device 100 may determine whether an attachment is necessary based on whether the checker orthodontic force is present or whether the checker orthodontic force corresponds to the crown orthodontic force.

A case in which the checker orthodontic force corresponds to the crown orthodontic force may occur, for example, when the checker orthodontic force can be substituted with the crown orthodontic force. The case in which the checker orthodontic force can be substituted with the crown orthodontic force may occur, for example, when the orthodontic force corresponding to the labial or buccal surface of the checker 310 corresponds to the crown orthodontic force. If the checker orthodontic force can be substituted with the crown orthodontic force, it may be interpreted that normal tooth movement is achievable using only the crown orthodontic force without the need for the checker orthodontic force. In this case, the device 100 may determine that an attachment is unnecessary.

However, if the checker orthodontic force cannot be substituted with the crown orthodontic force, it may be interpreted that the desired tooth movement cannot be achieved with only the orthodontic force applied to the crown. In this case, the device 100 may determine that an attachment is necessary. A case where the checker orthodontic force cannot be substituted with the crown orthodontic force may occur, for example, when the orthodontic force corresponding to the edge-adjacent region of the checker 310 is not applied to the crown 320 and thus cannot be replaced by the crown orthodontic force. According to one embodiment, the orthodontic force may result from the pressure between an orthodontic device and the crown 320, and the orthodontic device may apply the orthodontic force to the crown 320.

Specifically, if there is orthodontic force corresponding to the edge-adjacent region of the checker 310 or if orthodontic force is greater than or equal to a preset level, the device 100 may determine that an attachment is necessary. Additionally, the device 100 may determine that an attachment is necessary if there is orthodontic force corresponding to the checker 310 but no crown orthodontic force.

Conversely, the device 100 may determine that an attachment is unnecessary when there is no orthodontic force corresponding to the edge-adjacent region of the checker 310, or the orthodontic force is below the preset level, when there is no orthodontic force corresponding to the edge-adjacent portion of the checker 310, or the orthodontic force is below the preset level, while the crown orthodontic force is at or above the preset level, when orthodontic force is present only on surfaces other than the edge-adjacent region of the checker 310 (e.g., the labial or buccal surface of the checker 310, or when orthodontic force is present only on surfaces other than the edge-adjacent region of the checker 310, while the crown orthodontic force is at or above the preset level.

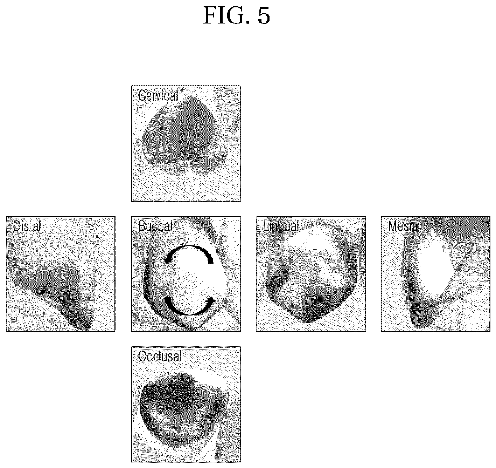

Detailed explanations of related embodiments will be provided below with reference to FIGS. 5 to 12.

In step S250, the device 100 may display the attachment-related information.

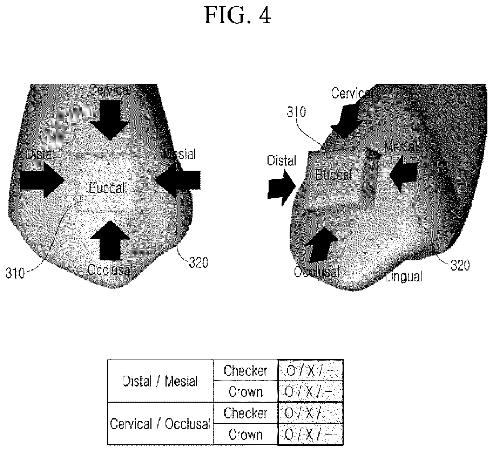

The attachment-related information may include information indicating whether an attachment is necessary and information indicating the basis for the necessity of the attachment, and may be displayed in the form of an attachment example model 305 shown in FIG. 3 and as tables (or text) shown in FIGS. 4, 6, 8, 10, and 12.

For example, the device 100 may first define the attributes of the attachment based on the attachment-related information and acquire the checker orthodontic force data that can be presented based on the orthodontic force data. The attributes of the attachment may include the orthodontic force acting on the checker 310, the attachment direction during attachment to the checker 310, whether size is adjusted during attachment to the checker 310, and whether the direction is changed.

Second, the device 100 may visualize the degree of difference before and after tooth movement due to orthodontic treatment (crown) by converting numerical information about movement distance difference into a specified color and displaying it on the pre-movement tooth model. Furthermore, the embodiments described in this disclosure are characterized by utilizing the distance difference after tooth movement as the pushing force to implement tooth movement, based on the principle of a removable orthodontic device applying pushing forces to teeth. A detailed description of the embodiment visualizing the degree of difference before and after tooth movement will be provided below with reference to the orthodontic force data 304 in FIG. 3.

Third, since the device 100 needs to detect a tooth and its region where the orthodontic force required to provide the pushing force for tooth movement is not exerted, it is characterized by using an additional checker (e.g., for orthodontic force evaluation) on the tooth. The device 100 may examine the orthodontic force reflected on the additional checker along with the orthodontic force acting on the corresponding tooth to ultimately determine the necessity of the attachment.

Fourth, the device 100 may propose specific attachments when necessary. For example, to ensure that planned removable orthodontic treatment is properly manifested within the oral cavity while simultaneously maintaining the advantages of a convenient treatment process, the device 100 may present predefined attachments limited to teeth requiring reinforcement of orthodontic force and place the attachments at the positions on individual teeth.

In this way, orthodontic force obtained through a comparison of pre- and post-orthodontic tooth models may be used to automatically provide the attachment-related information required for orthodontic treatment using a clear aligner. Additionally, by providing whether an attachment is necessary and the basis for this decision, and allowing the user to review it, the determination of whether an attachment is necessary may be made more easily.

FIG. 3 schematically illustrates an example in which an attachment example model 305 is displayed according to one embodiment. According to one embodiment, an attachment 330 may be attached to a crown or the like to increase the orthodontic force applied to the crown or the like.

Referring to FIG. 3, the device 100 may acquire a pre-orthodontic tooth model 301 and a post-orthodontic tooth model 302 and obtain a matched model 303 by matching the pre-orthodontic tooth model 301 and the post-orthodontic tooth model 302 based on a preset reference point.

Subsequently, the device 100 may obtain a comparison result for the pre- and post-orthodontic tooth models 301 and 302 from the matched model 303 based on the extent of movement of the crown 320 and the checker 310 before and after orthodontic treatment. Based on the obtained comparison result, the device 100 may acquire the crown orthodontic force and the checker orthodontic force.

The device 100 may acquire the attachment-related information based on the acquired crown orthodontic force and checker orthodontic force, and based on this, it may acquire and display an attachment example model 305.

For example, if the attachment-related information determines that the attachment 330 is necessary for the first crown 340, determines that there is checker orthodontic force for a predefined direction corresponding to the first crown 340 or that the checker orthodontic force is at or above a preset level, and determines that there is no crown orthodontic force for the predefined direction corresponding to the first crown 340 or that the crown orthodontic force is below the preset level, the device 100 may acquire the attachment example model 305 in which attachments corresponding to the positions and number of the checker orthodontic forces are attached to the first crown 340, and may display the attachment example model 305.

FIG. 4 schematically illustrates an example in which whether an attachment is required for orthodontic treatment is determined based on the orthodontic force applied in each direction to the checker 310 and the crown 320 according to one embodiment.

Referring to FIG. 4, the device 100 may determine the attachment-related information, including whether an attachment is necessary and the basis for its necessity, based on whether there is orthodontic force applied to the checker 310 and the crown 320 in preset directions such as distal, mesial, cervical, and occlusal directions, and the magnitude of the orthodontic force. The device 100 may display this information in the form of a table or text.

For example, the device 100 may determine the orthodontic force applied to the checker 310 and the crown 320 in the distal and mesial directions, as well as the orthodontic force applied to the checker 310 and the crown 320 in the cervical and occlusal directions, and provide attachment-related information based on whether orthodontic force is applied to the checker 310 and the crown 320 in each direction and the magnitude of the orthodontic force.

Detailed embodiments related to this will be explained with reference to FIGS. 5 to 12 below.

FIGS. 5 to 12 schematically illustrate examples in which whether an attachment is necessary is determined based on the orthodontic force applied in each direction to the checker 310 and the crown 320 according to one embodiment.

Specifically, FIGS. 5, 7, 9, and 11 illustrate the distribution and magnitude (or movement distance due to orthodontic treatment) of the crown orthodontic force applied in each direction to the crown 320, as well as the curvature of the crown orthodontic force in each direction.

In one embodiment, the curvature of the crown orthodontic force may be determined based on how uniform the distance is between the orthodontic force distribution area, which is an area where the orthodontic force is applied on the surface of the crown 320, and the tooth axis. For example, the curvature of the crown orthodontic force when the distance between the orthodontic force distribution area and the tooth axis is uniform may be greater than when the distance between the orthodontic force distribution area and the tooth axis is not uniform. As the curvature of the crown orthodontic force increases, the orthodontic device is more likely to slip on the surface of the crown 320. Therefore, the necessity of an attachment may be determined based on the curvature of the crown orthodontic force. For instance, if the curvature of the crown orthodontic force is greater than or equal to a preset value, it may be determined that an attachment is necessary.

In addition, FIGS. 6, 8, 10, and 12 illustrate the checker 310 attached to the crown 320 and attachment-related information, which includes the distribution of the checker orthodontic force on the checker 310, the magnitude of the checker orthodontic force (or movement distance due to orthodontic treatment), information indicating the basis for whether an attachment is necessary, and information indicating whether an attachment is required, in addition to the information shown in FIGS. 5, 7, 9, and 11.

Moreover, the information indicating the basis for whether an attachment is necessary, as shown in FIGS. 6, 8, 10, and 12, may be displayed in a table on one side of the drawings.

Specifically, in a case where checker orthodontic force greater than or equal to a preset reference value occurs in a region on the checker 310 corresponding to at least one direction for the checker 310, the possibility that an attachment may be necessary may be indicated with an "O" in the table. Conversely, if the checker orthodontic force below the reference value occurs, the possibility that an attachment may be unnecessary may be indicated with an "X." For example, when the checker orthodontic force greater than or equal to the reference value occurs, orthodontic treatment using an attachment is possible, and thus whether an attachment is required may depend on the crown orthodontic force. However, if the checker orthodontic force is below the reference value, orthodontic treatment using an attachment is not possible, and thus the attachment may be deemed unnecessary.

Additionally, the diagram may indicate with an "X" that an attachment is unnecessary in cases where crown orthodontic force occurs in the same direction as the direction in which the checker orthodontic force occurs, the crown orthodontic force is distributed across the entire area of the crown 320 in that direction, and it is unaffected by adjacent teeth or gums. For example, if the crown orthodontic force is distributed across the entire area of the crown 320, the curvature of the crown orthodontic force is low, meaning that the orthodontic device is unlikely to slip due to curvature, allowing normal orthodontic treatment without the need for an attachment, and thus the attachment may be unnecessary.

Additionally, in cases where crown orthodontic force occurs in the same direction as the direction in which the checker orthodontic force occurs, the crown orthodontic force is distributed only over a partial area of the crown 320 in that direction, the curvature of the crown orthodontic force is small (e.g., when the distance between the outer edge of the crown orthodontic force area and the tooth axis is not uniform), and the crown orthodontic force is unaffected by adjacent teeth, the determination of whether an attachment is necessary may not apply, which can be indicated with a "-" in the table. For example, when the crown orthodontic force is distributed only over a partial area of the crown 320, the crown orthodontic force may be weak, or it may not be applied to the required position for orthodontic treatment, in which case an attachment may be necessary. However, if the curvature of the crown orthodontic force is small, the orthodontic device may not slip, allowing the crown orthodontic force to be properly applied to the crown 320, and an attachment may not be necessary. Therefore, in such cases, the determination of whether an attachment is necessary may be excluded, and the necessity of an attachment may be determined based on the determination of the necessity of an attachment according to the checker orthodontic force and crown orthodontic force from other perspectives.

Additionally, in other cases (e.g., when crown orthodontic force is absent, or when crown orthodontic force is not transmitted due to interference from adjacent teeth or gums), the need for an attachment may be indicated with an "O."

In the following description, FIGS. 5 to 12 will be explained with reference to the above descriptions.

FIG. 5 is a diagram illustrating that orthodontic force is required in the direction of arrows shown from the buccal perspective according to the crown orthodontic force applied in each direction of the crown 320. The orthodontic force may represent a counterclockwise rotational force from the buccal perspective.

FIG. 6 is a diagram that additionally illustrates the checker 310 and the checker orthodontic force, based on the diagram shown in FIG. 5. Referring to FIG. 6, it is shown that the counterclockwise rotational orthodontic force shown on the crown 320 from the buccal perspective is applied to correspond to the checker orthodontic force applied to the edge-adjacent regions of the checker 310 (the left and bottom edge-adjacent regions of the checker 310 from the buccal perspective). For reference, in FIG. 6, the edge-adjacent regions of the checker 310 may represent the distal region 610, the mesial region 620, the cervical region 630, and the occlusal region 640, respectively.

Referring to the table for explanation, since the checker orthodontic force in the distal direction, among the distal and mesial directions, is at or above the preset level, the device 100 may indicate this with an "O" in the corresponding cell of the table. Additionally, since the checker orthodontic force in the occlusal direction, among the cervical and occlusal directions, is at or above the preset level, the device 100 may indicate this with an "O" in the corresponding cell of the table.

Additionally, the crown orthodontic force in the distal direction, among the distal and mesial directions, is not applied to the crown 320 due to interference from adjacent teeth, while the crown orthodontic force in the mesial direction is applied only to part of the area and exhibits high curvature. Therefore, the device 100 may indicate this with an "O" in the corresponding cell of the table. Furthermore, the crown orthodontic force in the cervical direction, among the cervical and occlusal directions, is not applied to the crown due to interference from the gums, while the crown orthodontic force in the occlusal direction exhibits high curvature. Thus, the device 100 may indicate this with an "O" in the corresponding cell of the table.

As a result, based on the determined results for the crown orthodontic force and checker orthodontic force shown in FIGS. 5 and 6, the device 100 may determine that an attachment is necessary for the crown 320 illustrated in FIGS. 5 and 6 and may provide information indicating that an attachment is required, along with the table.

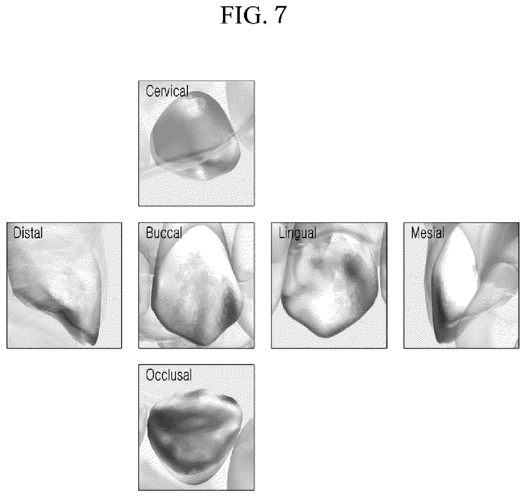

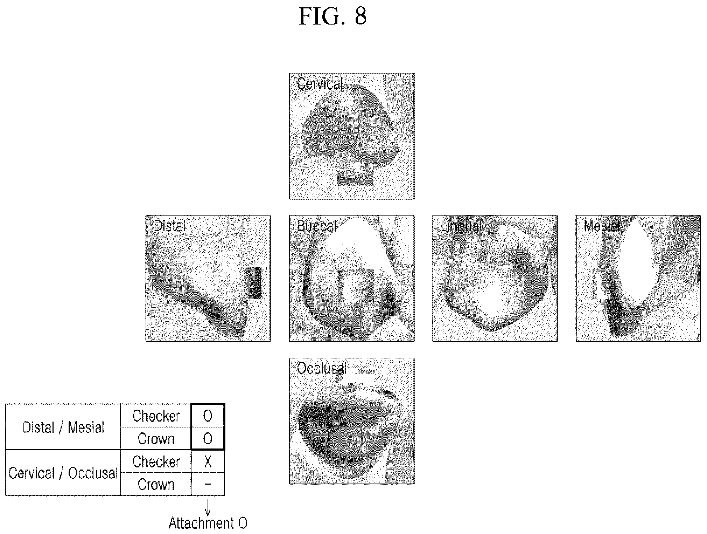

FIGS. 7 and 8 are diagrams illustrating examples where an attachment is necessary for the crown 320 shown in the drawings.

Referring to FIGS. 7 and 8, the checker orthodontic force in the distal and mesial directions is at or above the preset level. Therefore, the device 100 may indicate this with an "O" in the corresponding cell of the table. Additionally, since the checker orthodontic force in the cervical and occlusal directions is below the preset level, the device 100 may indicate this with an "X" in the corresponding cell of the table.

In addition, the crown orthodontic force in the distal direction, among the distal and mesial directions, is below the preset level and is not applied to the crown 320 due to interference from adjacent teeth, while the crown orthodontic force in the mesial direction is also below the preset level. Therefore, the device 100 may indicate this with an "O" in the corresponding cell of the table. Furthermore, the crown orthodontic force in the cervical direction, among the cervical and occlusal directions, is not applied to the crown 320 due to interference from the gums, while the crown orthodontic force in the occlusal direction is distributed only in part of the crown's area. Thus, the device 100 may indicate this with a "-" in the corresponding cell of the table.

As a result, the device 100 may determine that an attachment is necessary for the crown 320 based on the information displayed in the table, which provides the basis for the necessity of the attachment.

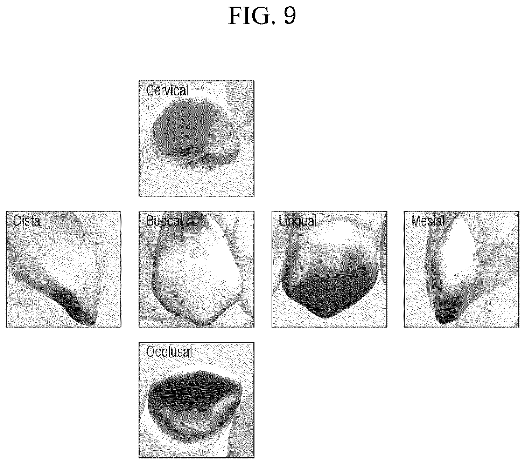

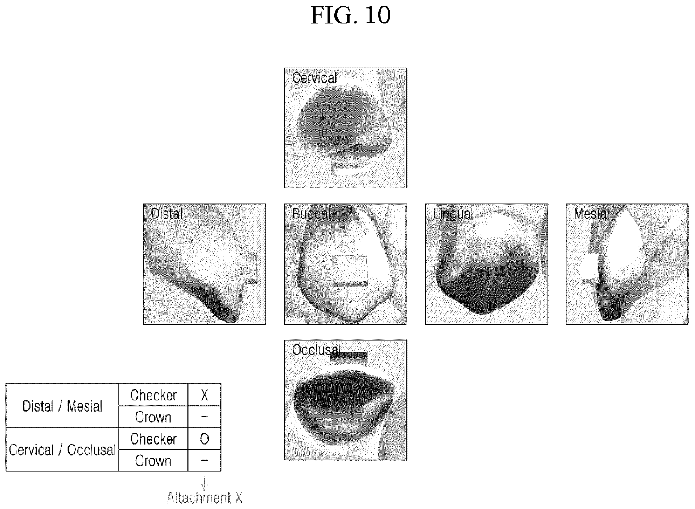

FIGS. 9 and 10 are diagrams illustrating examples where an attachment is unnecessary for the crown 320 shown in the drawings.

Referring to FIGS. 9 and 10, the checker orthodontic force in the distal and mesial directions is below the preset level. Therefore, the device 100 may indicate this with an "X" in the corresponding cell of the table. Additionally, since the checker orthodontic force in the occlusal direction, among the cervical and occlusal directions, is at or above the preset level, the device 100 may indicate this with an "O" in the corresponding cell of the table.

Moreover, the crown orthodontic force in the distal direction, among the distal and mesial directions, is applied to only part of the crown 320 at a level below the preset level, and most of the crown orthodontic force is not applied to the crown 320 due to interference from adjacent teeth. Similarly, the crown orthodontic force in the mesial direction is also applied to only part of the crown 320 at a level below the preset level. Therefore, the device 100 may indicate this with a "-" in the corresponding cells of the table.

Additionally, the crown orthodontic force in the cervical direction, among the cervical and occlusal directions, is not applied to the crown 320 due to interference from the gums, while the crown orthodontic force in the occlusal direction is applied so that it is distributed across the entire area of the crown 320. Therefore, the device 100 may indicate this with an "X" in the corresponding cell of the table.

As a result, the device 100 may determine that an attachment is unnecessary for the crown 320 based on the information displayed in the table, which provides the basis for the necessity of the attachment.

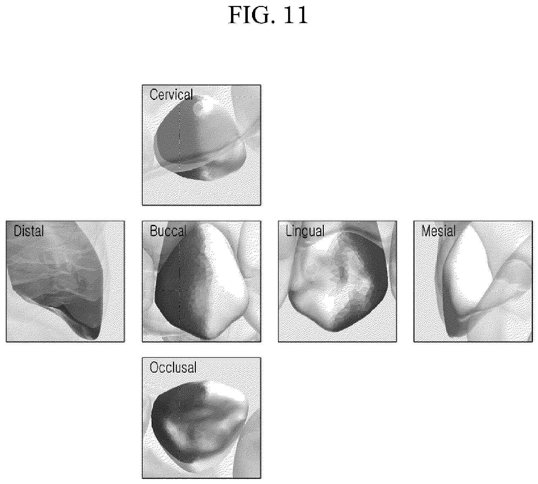

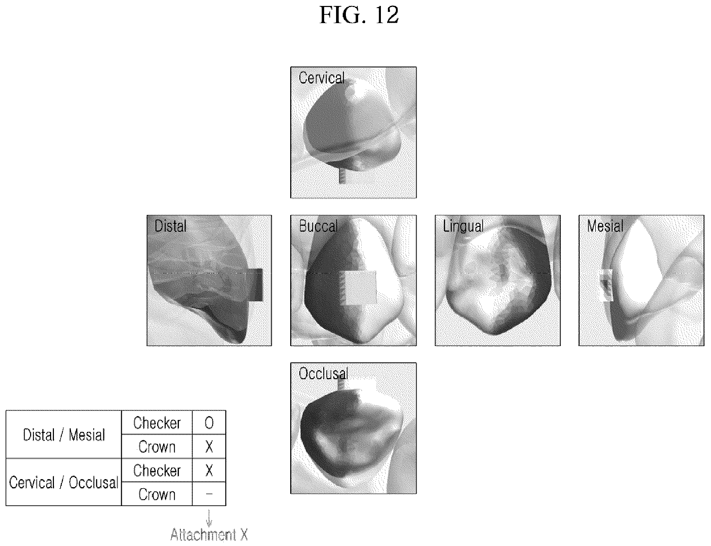

FIGS. 11 and 12 are diagrams illustrating examples where an attachment is unnecessary for the crown 320 shown in the drawings.

Referring to FIGS. 11 and 12, since the checker orthodontic force in the distal direction, among the distal and mesial directions, is at or above the preset level, the device 100 may indicate this with an "O" in the corresponding cell of the table. Additionally, since the checker orthodontic force in the cervical and occlusal directions is below the preset level, the device 100 may indicate this with an "X" in the corresponding cell of the table.

Furthermore, the crown orthodontic force in the distal direction, among the distal and mesial directions, is applied to the entire area of the crown 320 at or above the preset level, and because the area where the crown orthodontic force is applied is relatively large, the influence from adjacent teeth is considered minimal. Therefore, the device 100 may indicate this with an "X" in the corresponding cell of the table. Additionally, the crown orthodontic force in the cervical direction, among the cervical and occlusal directions, is not applied to the crown 320 due to interference from the gums, while the crown orthodontic force in the occlusal direction is applied to only part of the crown 320. Thus, the device 100 may indicate this with a "-" in the corresponding cell of the table.

Moreover, since the checker orthodontic force applied in the buccal direction is also present at a certain level, the crown 320 may not require an attachment.

As a result, the device 100 may determine that an attachment is unnecessary for the crown 320 based on the information displayed in the table, which provides the basis for the necessity of the attachment.

**In** this way, the device 100 may determine the basis information for the necessity of an attachment based on the orthodontic force applied in each direction to the checker 310 and the crown 320, and determine and provide whether an attachment is necessary based on this basis information. By allowing the user to review the provided information, the determination of whether an attachment is necessary may be made more easily.

It should be appreciated that the order and combination of the steps shown above is merely an embodiment of the present disclosure, and the order, combination, branch, function and the performing subject may vary to be implemented with addition, fewer, or different steps without departing from the essential characteristics of each component described in the specification. Throughout this specification, the term "provide (or providing)" may be interpreted as comprehensively including a process in which an object obtains specific information or directly or indirectly transmits or receives specific information to or from a specific object and including the performance of related operations required in this process.

Various embodiments set forth herein may be implemented as software comprising one or more instructions stored in a storage medium (e.g., memory) that is readable by a machine (e.g., a display device or a computer). For example, a processor (e.g., the processor 120) of the machine may invoke at least one of the one or more instructions stored in the storage medium, and execute it. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

It will be understood by one of ordinary skill in the art that various changes in form and details may be made therein without departing from the spirit and scope of the exemplary embodiments as defined by the following claims. Thus, the disclosed methods should be considered in a descriptive sense only and not for purposes of limitation. The scope of the present disclosure is presented not in the foregoing description but in the claims and all differences within an equivalent range thereto should be construed as being included in the present disclosure

## Claims

1. A method performed by a device for providing information for an orthodontic attachment, the method comprising the steps of:
acquiring a pre-orthodontic tooth model indicating a pre-orthodontic state of a tooth model to which a virtual checker is attached;
acquiring a post-orthodontic tooth model indicating a post-orthodontic state of the tooth model;
acquiring orthodontic force data based on a comparison result of the pre-orthodontic tooth model to the post-orthodontic tooth model;
acquiring attachment-related information including information on whether an attachment is required for orthodontic treatment, based on checker orthodontic force data, which represents orthodontic force corresponding to the checker and is acquired from the orthodontic force data; and
displaying, at an output part, the attachment-related information.

2. The method of claim 1, wherein the step of acquiring the attachment-related information comprises determining whether the attachment is necessary based on whether there is the orthodontic force corresponding to the checker.

3. The method of claim 1, wherein the step of acquiring the attachment-related information comprises the steps of:
acquiring crown orthodontic force data from the orthodontic force data, wherein the crown orthodontic force data represents orthodontic force corresponding to a crown of the tooth model that includes the crown and the checker; and
acquiring the attachment-related information using both the checker orthodontic force data and the crown orthodontic force data.

4. The method of claim 3, wherein the step of acquiring the attachment-related information using both the checker orthodontic force data and the crown orthodontic force data comprises the steps of:
determining checker orthodontic force, which is orthodontic force corresponding to the checker, based on the checker orthodontic force data;
determining crown orthodontic force, which is orthodontic force corresponding to the crown, based on the crown orthodontic force data; and
determining whether the attachment is necessary based on whether the checker orthodontic force corresponds to the crown orthodontic force.

5. The method of claim 4, wherein the step of determining whether the attachment is necessary based on whether the checker orthodontic force corresponds to the crown orthodontic force comprises determining that the attachment is unnecessary when the checker orthodontic force is substitutable with the crown orthodontic force, and determining that the attachment is necessary when the checker orthodontic force is not substitutable with the crown orthodontic force.

6. The method of claim 1, wherein the step of acquiring the attachment-related information comprises the steps of:
determining a direction of the orthodontic force based on a magnitude and distribution of the orthodontic force corresponding to the checker; and
acquiring the attachment-related information based on the direction of the orthodontic force.

7. The method of claim 1, wherein the step of acquiring the attachment-related information comprises determining whether the attachment is necessary based on whether there is orthodontic force corresponding to an edge-adjacent region of the checker having an angular shape.

8. The method of claim 1, wherein the checker is attached to the buccal or labial side of a crown and has a rectangular shape including a first edge-adjacent region to a fourth edge-adjacent region, and
wherein the step of acquiring the attachment-related information comprises the steps of: determining a direction of the orthodontic force to be at least one of a distal direction, a mesial direction, a cervical direction, or an occlusal direction based on a region to which the orthodontic force corresponds, among the first edge-adjacent region to the fourth edge-adjacent region; and
acquiring the attachment-related information based on the direction of the orthodontic force.

9. The method of claim 1, wherein the step of acquiring the orthodontic data comprises determining a region to which the orthodontic force corresponds and a magnitude of the orthodontic force based on the degree of separation between the pre-orthodontic tooth model and the post-orthodontic tooth model when they are matched.

10. The method of claim 1, wherein the orthodontic force data includes data on a magnitude of the orthodontic force and data on a distribution of the orthodontic force.

11. A device for providing information for an orthodontic attachment, comprising:
a reception part configured to acquire a pre-orthodontic tooth model indicating a pre-orthodontic state of a tooth model to which a virtual checker is attached and a post-orthodontic tooth model indicating a post-orthodontic state of the tooth model;
a processor configured to
acquire orthodontic force data based on a comparison result of the pre-orthodontic tooth model to the post-orthodontic tooth model and
acquire attachment-related information including information on whether an attachment is required for orthodontic treatment, based on checker orthodontic force data, which represents orthodontic force corresponding to the checker and is acquired from the orthodontic force data; and
an output part configured to display the attachment-related information.

12. The device of claim 11, wherein the processor is configured to
acquire crown orthodontic force data from the orthodontic force data , wherein the crown orthodontic force data represents orthodontic force corresponding to a crown of the tooth model that includes the crown and the checker, and
acquire the attachment-related information using both the checker orthodontic force data and the crown orthodontic force data.

13. The device of claim 12, wherein the processor is configured to
determine checker orthodontic force, which is orthodontic force corresponding to the checker, based on the checker orthodontic force data,
determine crown orthodontic force, which is orthodontic force corresponding to the crown, based on the crown orthodontic force data, and
determine whether the attachment is necessary based on whether the checker orthodontic force corresponds to the crown orthodontic force.

14. The device of claim 13, wherein the processor is configured to
determine that the attachment is unnecessary when the checker orthodontic force is substitutable with the crown orthodontic force, and
determine that the attachment is necessary when the checker orthodontic force is not substitutable with the checker orthodontic force.

15. The device of claim 11, wherein the processor is configured to determine whether the attachment is necessary based on whether there is orthodontic force corresponding to an edge-adjacent region of the checker having an angular shape.

16. A computer-readable recording medium having recorded thereon a program which, when executed by a computer, causes the computer to perform the method of any one of claims 1 to 10.
